# EUROPEAN PATENT APPLICATION

(11) **EP 1 523 979 A1**
(43) Date of publication of application: **20.04.2005**
(21) Application number: 03256438.7
(22) Date of filing: 13.10.2003
(51) Int. Cl.: A61K 9/24, A61K 31/137

(54) **Extended release pharmaceutical dosage form**

(71) Applicant: Wyeth, Madison, New Jersey 07940 (US)
(72) Inventor: Heaton, Nicholas, Altrincham Cheshire WA15 7NP (GB); Potts, Angela, Titchfield Hampshire PO14 4NT (GB); Armstrong, Ian, Waltham Chase Hampshire, SO32 2NA (GB); Provost, James Andrew, Waltham Chase Southampton (GB)
(74) Representative: Wileman, David Francis Dr.

(57) **Abstract**

This invention relates to novel extended release pharmaceutical dosage forms for orally delivering drugs to mammals, e.g., humans. More particularly, this invention concerns novel dosage forms of water soluble drugs such as venlafaxine, enantiomeric (R or S) forms of venlafaxine, metabolites of venlafaxine such as O-desmethyl venlafaxine (ODV) or enantiomeric (R or S) forms of said metabolites which dosage forms have an extended release profile when taken orally. This invention also provides processes for preparing such dosage forms and methods of using them.

## Description

This invention relates to novel extended release pharmaceutical dosage forms for orally delivering drugs to animals, e.g., mammals such as humans or dogs. More particularly, this invention concerns novel dosage forms of water soluble drugs such as venlafaxine, enantiomeric (R or S) forms of venlafaxine, metabolites of venlafaxine such as O-desmethyl venlafaxine (ODV) or enantiomeric (R or S) forms of said metabolites which dosage forms have an extended release profile when taken orally. This invention also provides processes for preparing such dosage forms and methods of using them.

### Background of the Invention

Venlafaxine, 1-[2-(dimethylamino)-1-(4-methoxyphenyl)ethyl]-cyclohexanol, is an important drug in the neuropharmacological armentarium used for treatment of depression and general anxiety disorders. Venlafaxine and the acid addition salts thereof are disclosed in US patent 4,535,186. Venlafaxine as the hydrochloride salt is presently administered orally to adults in compressed tablet form taken two or three times a day within the range 75 to 350 mg/day. EP 0639374 discloses the use of venlafaxine in the treatment of obesity, panic disorder, post-traumatic stress disorder, late luteal phase dysphoric disorder attention deficit disorder, Gilles de la Tourette syndrome, bulimia nervosa, generalised anxiety disorder or Shy Drager syndrome. EP-A-654264 teaches the use of venlafaxine in treating incontinence. U.S. Patent No. 5,506,270 (Upton et al.) claims venlafaxine's use in methods of treating hypothalamic amenorrhea in non-depressed women. US Patent No. 5,530,013 (Husbands et al.) claims venlafaxine's use for enhancing cognition.

N-desmethylvenlafaxine (NDV) and O-desmethylvenlafaxine (ODV) are active metabolites of venlafaxine that can be produced as described in Example 12 and 26 respectively of U.S. Patent No. 4,535,186 (Husbands et al.), the entirety of which is incorporated herein by reference. It will be understood that the enantiomers may be separated from each other by standard resolution techniques known in the art. An example of such resolution techniques is that described by Yardley et al. for resolution of 1-[2-(Dimethylamino)-1-(4-methoxyphenyl)ethyl]cyclohexanol in J. Med. Chem, 1990, Vol. 33, No. 10, at page 2904. ,

The absolute configuration of the (+) enantiomer of venlafaxine was established as S by a single crystal X-ray analysis of the hydrobromide salt and the anomalous dispersion technique (Yardley et al., J. Med. Chem., 1990, 33, 2899).

WO 00/32335 and WO 00/32336 respectively teach derivatives of (+)- and (-)-venlafaxine. WO 00/59851 teaches derivatives of venlafaxine.

WO 00/76955 teaches the pharmaceutically active enantiomers of the venlafaxine metabolite O-desmethyl venlafaxine, R(-)-4-[2-(dimethylamino-1-(1-hydroxycyclohexyl)ethyl]phenol and S(+)-4-[2-(dimethylamino-1-(1-hydroxy-cyclohexyl)ethyl]phenol useful in treating depression

WO 00/76956 teaches the pharmaceutically active enantiomers of the venlafaxine metabolite N-desmethyl venlafaxine, (S)-1-[1-(4-methoxyphenyl)-2-(methylamino)ethyl]cyclohexanol and (R)-1-[1-(4-methoxyphenyl)-2-(methylamino)ethyl]cyclohexanol useful in treating depression.

U.S. Patents Nos. 5,788,986 (Dodman) and 5,554,383 (Dodman) teach and claim the veterinary use of serotonin reuptake inhibitors including (±)-, R- or S-venlafaxine or active metabolites thereof including, desmethylvenlafaxine, 4-hydroxyvenlafaxine and didesmethylvenlafaxine, in modifying the behavior of dogs.

In therapeutic dosing with venlafaxine hydrochloride immediate release tablets, rapid dissolution results in a rapid increase in blood plasma levels of the active compound shortly after administration followed by a decrease in blood plasma levels over several hours as the active compound is eliminated or metabolized, until sub-therapeutic plasma levels are approached after about twelve hours following administration, thus requiring additional dosing with the drug. With the plural daily dosing regimen, the most common side effect is nausea, experienced by about forty five percent of patients under treatment with venlafaxine hydrochloride (see Entsuahi, R. and Chitra, R. Psychopharm. Bul., Vol. 33(4) 671 1997). Vomiting also occurs in about seventeen percent of the patients.

To ameliorate the problem encapsulated sustained release formulations of venlafaxine hydrochloride have been developed; - see EP 0797991A1 published 1 October 1997 which discloses encapsulated venlafaxine sustained release formulations wherein microcrystalline cellulose and hydroxypropylmethylcellulose were used in making practical venlafaxine-containing spheroids. EP 0797991 A1 states that it was completely unexpected that an extended release formulation containing venlafaxine hydrochloride could be obtained because the hydrochloride of venlafaxine proved to be extremely water soluble.

EP 0797991 A1 further states that numerous spheroid formulations were prepared using different grades of microcrystalline cellulose and hydroxypropyl methylcellulose, different ratios of venlafaxine hydrochloride and filler, different binders such as polyvinylpyrrolidone, methylcellulose, water, and polyethylene glycol of different molecular weight ranges in order to find a formulation which would provide a suitable granulation mix which could be extruded properly. In the extrusion process, heat buildup occurred which dried out the extrudate so much that it was difficult to convert the extruded cylinders into spheroids. EP 0797991 A1 then states further that addition of hydroxypropylmethylcellulose 2208 to the venlafaxine hydrochloride-microcrystalline cellulose mix made production of spheroids practical.

WO 99/22724 published 14 May 1999 discloses an encapsulated extended release venlafaxine formulation comprising spheroids substantially free of hydroxypropylmethylcellulose.

WO 94/27589 published 8 December 1994 discloses an osmotic dosage form that delivers a drug by osmotic action over an extended period of time. The drug composition comprised of venlafaxine and hydroxypropylalkylcellulose is delivered by displacement composition.

Immediate release venlafaxine hydrochloride tablets are marketed by Wyeth-Ayerst Laboratories under the Effexor® trademark. An extended release formulation of venlafaxine hydrochloride salt is available as Effexor XR for use in humans which is in the form of a capsule (37.5, 75mg and 150 mg) for once daily dosing, typically in the range from 75 mg/day to 225 mg/day. From pharmacokinetic studies the bioavailability of venlafaxine from such formulations is in the order of 40-45%, (see Patat et al., J. Clin. Pharmacol., 38 256 1998). Furthermore the rate of release over an extended period shows some variation and there is a need therefore for a more consistent release profile. Additionally such an encapsulated formulation is more difficult and more time consuming to manufacture than extended release tablets which would require less sophisticated machinery.

There is a need for an extended release tablet formulation of a highly water soluble drug such as venlafaxine hydrochloride.

There is a need for an extended release formulation of a drug with a substantially linear release profile, i.e. approximating to zero order characteristics, to provide a steady release of active for an extended period.

There is also a need for an extended release formulation of venlafaxine hydrochloride which is simpler to manufacture than the filled capsule formulation.

We have surprisingly found that it is possible to prepare a sustained release pharmaceutical or veterinary formulation in tablet form which has an approximately zero order release profile of the active drug combined with satisfactory drug bioavailability; and which is especially suitable for a highly water soluble drug such as a venlafaxine salt, e.g., hydrochloride or a derivative thereof.

Furthermore we have been able to prepare such a tablet with satisfactory physical properties for bulk manufacture and commercial use.

Accordingly, this invention provides an oral pharmaceutical or veterinary dosage form for extended release of a biologically active drug that is soluble in gastrointestinal media, the dosage form comprising:
(i) a solid core formed from a melt of a first substance which is substantially insoluble in gastrointestinal media and having dispersed therein the active drug, and
(ii) a coating applied to the core from a solution or melt followed by drying or cooling as appropriate, the coating comprising a second substance which is insoluble in gastrointestinal media and a component which is soluble in gastrointestinal media.

Preferably, each of the first and second substances is a wax. Still more preferably, each of the first and second substance is a hydrophobic wax such as one or more of stearic acid, hydrogenated vegetable oil, glyceryl monostearate or cetostearyl alcohol. Most preferably the wax is stearic acid.

Typically the core may comprise from about 25% to about 75% (w/w) of the first substance, from about 75% to about 25% (w/w) of the active drug and from 0 to 50% (w/w) of filler or excipient such as diluents, glidants or lubricants or any combination thereof.

In some embodiments the core may comprise from about 35% to about 60% (w/w) of the first substance, from about 60% to about 35% (w/w) of the active drug and from 5 to 35% (w/w) of filler.

Preferably the core comprises from about 45% to about 50% (w/w) of the first substance, from about 43% to about 38% (w/w) of the active drug and from 8 to 13% (w/w) of filler.

Typically the core comprises diluents, such as microcrystalline cellulose, glidants such as silicon dioxide, and lubricants, such as magnesium stearate.

Typically the coating comprises a second insoluble substance which may be the same as the first insoluble substance in the core and a soluble component which may be inert, e.g, polyethylene glycol, or may be a pharmaceutically active compound or a mixture of inert and active compound.

The ratio of insoluble to soluble material in the coat may be for example in the range from about 12:1 (w/w) to 1:10 (w/w). For soluble pharmaceutically active component the ratio may preferably be from about 6:1 to 4:1 (w/w) or about 5:1 (w/w). Typically for polyethylene glycol the ratio may be from about 10:1 to about 8:1 (w/w).

Preferably, the active pharmaceutical substance is venlafaxine, or a pharmaceutically acceptable salt, derivative or metabolite thereof. More preferably the active pharmaceutical substance is venlafaxine hydrochloride.

In another aspect, the present invention relates to a process for the preparation of a pharmaceutical dosage form as described above which comprises:
a) melting a mixture of the first insoluble substance and the active drug and solidifying to provide a core,
b) spray coating the core with a solution of the second substance and the soluble component or hot melt coating a melt of the second substance containing a dispersion of the soluble component; and
c) drying if appropriate.

Unless the context states otherwise, as used herein, reference to venlafaxine is to be taken to include reference to optical forms thereof, derivatives (including active matabolites) and optical forms of said derivatives. Similarly reference to 'pharmaceutical' or 'pharmaceutically', e.g., as used in the term pharmaceutically acceptable compositions or salts, is to be taken to include the veterinary equivalent.

Based on animal data the tablet formulations of this invention can provide, in a single daily dose, extended release of venlafaxine so that it is possible to maintain a steady state therapeutic blood serum level up to a twenty-four hour period. In particular through administration of the venlafaxine formulation of this invention, there is provided a method for obtaining a flattened drug plasma concentration to time profile, thereby affording a tighter plasma therapeutic range control than can be obtained with multiple daily dosing. Accordingly this invention provides a method for eliminating the sharp peaks and troughs (hills and valleys) in blood plasma drug levels induced by multiple daily dosing with conventional immediate release venlafaxine hydrochloride tablets. In essence, the plasma levels of venlafaxine hydrochloride rise, after administration of the extended release formulations of this invention, for about four hours and then begin to fall through a protracted, substantially linear decrease from the peak plasma level for the remainder of the twenty four hour period, maintaining at least a threshold therapeutic level of the drug during the entire twenty-four period. Hence, in accordance with the use aspect of this invention, there is provided a method for moderating the plural blood plasma peaks and valleys attending the pharmacokinetic utilization of multiple daily rapid release tablet dosing with venlafaxine hydrochloride which comprises administering to a patient in need of treatment with venlafaxine hydrochloride, a one-a-day, extended release pharmaceutical dosage form of the invention.

Resulting from the increase in bioavailability of venlafaxine in the compositions of this invention it is possible to lower the daily dosage amount of active which for capsule formulations was typically between 75 and 225 mg/day.

The active pharmaceutical ingredient may comprise about 10-90% w/w of the dosage form, e.g., about 20-50% w/w of the dosage form. Preferably the active comprises about 30-40% w/w of the dosage form. Examples of the active pharmaceutical ingredient in the fomulations of this invention include venlafaxine, (R)-venlafaxine, (S)-venlafaxine, (R)-O-desmethylvenlafaxine, (S)-O-desmethylvenlafaxine, or a highly water soluble salt thereof such as the hydrochloride acid salt.

In addition the core of the present invention can also comprise standard fillers or excipients including but not limited to one or more of talc, calcium carbonate, microcystalline cellulose, powdered cellulose, dextrates, kaolin, mannitol, silicic acid, sorbitol, starch, pre-gelatinized starch, dicalcium phosphate, microcrystalline cellulose, lactose and starch.

The dosage form may also comprise a lubricant. Suitable lubricants include, but are not limited to, calcium stearate, magnesium stearate, mineral oil, glycerin, sorbitol, mannitol, polyethylene glycol, other glycols, stearic acid, sodium lauryl sulfate, talc, hydrogenated vegetable oil, zinc stearate, ethyl oleate, ethyl laureate, agar, or mixtures thereof. Preferably, the lubricant magnesium stearate is used to aid core manufacture.

The coat of the present pharmaceutical dosage form, expressed as a proportion of the total weight of the dosage form, will vary according to the release characteristics required. This proportion will easily be determined through routine experimentation by one skilled in the art. Typically, the coat will constitute between 1 % and 20 % of the total weight of the pharmaceutical dosage form.

The ratio of soluble ingredient (e.g. active ingredient) to inert or insoluble material in the coat will also be determined by the release profile desired. A higher proportion of soluble ingredient will in general result in faster release. Routine experimentation by one skilled in the art will enable this ratio to be ascertained in any given case.

The ratio of active pharmaceutical ingredient to other ingredients in the core will also influence the release profile. Again, routine experimentation will enable the person skilled in the art to achieve a dosage form having the desired characteristics for any given application.

By varying the weight ratios of core to coat, and of the constituents of the core and coat, it is possible to obtain various dissolution profiles of active ingredient. Delayed release, burst release and zero-order release profiles are readily obtainable with the dosage forms of the present invention.

The cores of the present invention can be prepared by standard tableting procedures, such as for example, dry blending or melt granulation in accordance with methods known in the art. For instance, the active ingredient may be sieved through a suitable sieve and blended with excipients until a uniform blend if formed. The dry blend may be screened and blended with magnesium stearate. The resulting powder blend may then be compressed into tablets of desired shape and size. Preferred granulation and coating techniques are described below.

Coating of the cores may be achieved by standard techniques known in the art. In some circumstances, it will be advantageous to combine the inert material and the soluble ingredient prior to form a coating composition prior to coating the cores. Spray-coating is an example of a technique which may be used to apply the coat to the cores.

"Pharmaceutical dosage form" as used herein describes a solid entity used in administering an active pharmaceutical ingredient to a human or animal subject. Such dosage forms include tablets, pills, microspheres for inclusion in capsules, suppositories, pessaries, implants and the like.

"Core" as used herein means the nucleus of the pharmaceutical dosage form.

"Coating" and "coat" as used herein means the layer of the pharmaceutical dosage form covering substantially all of the core.

"Covering substantially all of the core" as used herein means covering more than 90 % of the core.

"Active pharmaceutical ingredient" as used herein means an ingredient eliciting a pharmacological effect.

"Matrix" as used herein means a continuous phase within which another component is dispersed.

"Inert material" as used herein means a component which does not illicit a physiological response.

"Soluble" and "substantially insoluble" refer to solubility in 0.9 % saline solution at 37 °C.

"Wax" as used herein refers to hydrocarbons and fatty acids or their esters solid at room temperature (at or about 20 °C).

The tablets of the present invention can be prepared by standard tableting procedures, such as for example, melt granulation for preparing the core and liquid coating of the cores in accordance with methods known in the art.

A preferred manufacturing process using venlafaxine HCl as the active drug is given below:
(a) Granulation
   Venlafaxine hydrochloride, stearic acid and microcrystalline cellulose are weighed and passed through a 500 µm screen into a granulator suitable for melt-granulation, and then processed until an even granule is formed. The granule is allowed to cool to ambient temperature and passed through a 1.4mm screen.
(b) Preparation of final blend
   the granule from (a) is blended with silicon dioxide in a suitable blender. Magnesium stearate is added, and blending is continued for approximately 1 minute.
(c) Preparation of tablets
   The blend from (b) is compressed to form tablets. The tablets are passed through a de-duster, metal checker and weight checker.
(d) Coating of tablets
   (1) Solvent Coating
      The soluble ingredient and stearic acid are dissolved in a suitable solvent, e.g. ethanol, and mixed until a clear solution is obtained.
   (2) Hot Melt Coating
      The soluble material is dispersed in molten stearic acid and mixed until evenly dispersed.

The tablets from step (c) were loaded into the coating pan of a tablet coater, and coating was continued until the average tablet weight is between the required limits. The tablets were allowed to cool, and visually inspected for defects.

The following examples illustrate this invention:

### EXAMPLE 1

An extended release tablet of venlafaxine hydrochloride containing 72mg (base) was made according to the melt-granulation manufacturing process described above with the following constituents below. The core was then coated using the solvent technique described above.

| **Tablet Core** | mg | % |
|---|---|---|
| Venlafaxine HCl (as base) | 81.45 (72) | 40.72 |
| Stearic acid | 96.55 | 48.28 |
| Microcrystalline cellulose | 20.00 | 10.00 |
| Colloidal silicon dioxide | 0.40 | 0.20 |
| Magnesium stearate | 1.60 | 0.80 |
| TOTAL core weight | 200.00 | |

| **Coating** | mg |
|---|---|
| Venlafaxine HCl (as base) | 3.39 (3.0) |
| Stearic acid | 16.97 |
| Total coating weight | 20.36 |
| TOTAL tablet weight | 220.36 |

### EXAMPLE 2

An extended release tablet of venlafaxine hydrochloride containing 72mg (base) was made according to the melt-granulation manufacturing process described above. The core was then coated using the solvent technique described above.

| **Tablet Core** | mg | % |
|---|---|---|
| Venlafaxine HCl (as base) | 81.45 (72) | 40.72 |
| Stearic acid | 96.55 | 48.28 |
| Microcrystalline cellulose | 20.00 | 10.00 |
| Colloidal silicon dioxide | 0.40 | 0.20 |
| Magnesium stearate | 1.60 | 0.80 |
| TOTAL core weight | 200.00 | |

| **Coating** | mg |
|---|---|
| Venlafaxine HCl (as base) | 3.39 (3.0) |
| Stearic acid | 20.36 |
| Total coating weight | 23.76 |
| TOTAL tablet weight | 223.76 |

### EXAMPLE 3

An extended release tablet of venlafaxine hydrochloride containing 72mg (base) was made according to the melt-granulation manufacturing process described above with the following constituents below. The core was then coated using the hot melt technique described above.

| **Tablet Core** | mg | % |
|---|---|---|
| Venlafaxine HCl (as base) | 81.45 (72) | 40.72 |
| Stearic acid | 96.55 | 48.28 |
| Microcrystalline cellulose | 20.00 | 10.00 |
| Colloidal silicon dioxide | 0.40 | 0.20 |
| Magnesium stearate | 1.60 | 0.80 |
| TOTAL core weight | 200 | |

| **Coating** | mg |
|---|---|
| Venlafaxine HCl (as base) | 3.39 (3.0) |
| Stearic acid | 16.41 |
| Total coating weight | 20.00 |
| TOTAL tablet weight | 220.00 |

### DISSOLUTION TESTING

Dissolution testing was carried out using USP Type 2 apparatus in 0.9 % saline at 100 rpm at 37 °C.

| Time (h) | % Dissolved | |
|---|---|---|
| | EXAMPLE 1 | EXAMPLE 3 |
| 1 | 2.6 | 3.7 |
| 2 | 6.6 | 6.3 |
| 4 | 14.9 | 12.5 |
| 8 | 29.4 | 25.6 |
| 12 | 43.8 | 38.6 |
| 18 | 66.6 | 58.6 |
| 24 | 80.5 | 78.3 |

A substantially linear release profile of venlafaxine HCl was obtained.

### (A) In vivo test results in dogs:

Each of 2 groups of 4 male beagle dogs received a 75mg dose of venlafaxine from the formulations above in fed state.
- Dogs 1-4 received commercial sustained release capsule formulation (Effexor XR), and the formulation of Example 1.
- Dogs 5-8 received sustained release venlafaxine HCl tablet formulation of Example 2.

| Mean venlafaxine bioavailability in dogs (n=4) | | | |
|---|---|---|---|
| Pharmacokinetic paramater | Formulation | | |
| | Effexor XR | Example 1 | Example 2 |
| AUC₀₋₂₄ (ng.h/ml) | 2130 | 3164 | 5715 |
| Cₘₐₓ (ng/ml) | 137 | 319 | 563 |
| Tₘₐₓ (h) | 5.5 | 6.8 | 10.5 |
| Relative Bioavailability^{a} | 31% | 47% | 84% |

| | | | |
|---|---|---|---|
| ^{a}Bioavailability relative to a solution dose taken from a previous dog study where the relative bioavailabilty of Effexor XR was shown to be 30% | | | |

| Mean free ODV bioavailability results in dogs (n=4) | | | |
|---|---|---|---|
| Pharmacokinetic paramater | Formulation | | |
| | Effexor XR | Example 1 | Example 2 |
| AUC₀₋₂₄ (ng.h/mf) | 216.8 | 175.3 | 296.8 |
| Cₘₐₓ (ng/ml) | 24.7 | 31.3 | 35.3 |
| Tₘₐₓ (h) | 2.5 | 1.8 | 7.0 |

These results show an increase in the bioavailability in dogs for the formulations of the present invention.

### (B) Human studies with formulation of Example 1 and Example 3

A 5-period crossover study was carried out with administration of Example 1 venlafaxine HCl/carbopol tablets and the marketed 75-mg venlafaxine XR capsule. Twenty (20) subjects enrolled in the study, and 19 subjects completed. One subject withdrew from the study after receiving the formulation of EXAMPLE 1, but did not receive the marketed XR capsule.

A second 4-period crossover study was carried out with administration of Example 3 venlafaxine HCl/carbopol tablets and the marketed 75-mg venlafaxine XR capsule. Twenty (20) subjects enrolled in the study.

The following two Tables summarize the pharmacokinetic profile of venlafaxine and its active metabolite, O-desmethylvenlafaxine for each formulation. The first line of the table relates to the first clinical study and the second line relates to the second clinical study.

| Mean Venlafaxine Bioavailability Results in Humans | | | |
|---|---|---|---|
| Pharmacokinetic parameter | FORMULATION | | |
| | Effexor XR | Example 1 | Example 3 |
| AUC₀₋₂₄ (ng.h/ml) | 729 | 586 | |
| | 658 | | 550 |
| Cₘₐₓ (ng/mL) | 38 | 26 | |
| | 36 | | 31 |
| tₘₐₓ (h) | 5.9 | 15.7 | |
| | 5.9 | | 12.1 |
| t_{1/2} (h) | 10.8 | 8.9 | |
| | 11.5 | | 10.1 |

| Mean Free ODV Bioavailability Results in Humans | | | |
|---|---|---|---|
| Pharmocokinetic parameter | Formulation | | |
| | Effexor XR | Example 1 | Example 3 |
| AUC₀₋₂₄ (ng.h/ml) | 2578 | 1978 | |
| | 2089 | | 1846 |
| Cₘₐₓ (ng/mL) | 83 | 63 | |
| | 77 | | 64 |
| tₘₐₓ (h) | 10.4 | 19.2 | |
| | 10.3 | | 16.6 |
| t_{1/2} (h) | 15.0 | 14.8 | |
| | 1.3 | | 11.4 |

These results demonstrate that the formulation of the invention have a Cₘₐₓ which is lower and occurs later than the marketed formulation Effexor XR

Nausea was the primary adverse event recorded in the two studies. The wax matrix formulations of the present invention cause less nausea than the commercial venlafaxine hydrochloride capsule, EFFEXOR XR. In the first study, Example 1 caused nausea in only 20% of patients compared with 37% for the marketed formulation. In the second study, Example 3 did not cause nausea compared with nausea in 10 % of patients with the marketed formulation. In terms of an explanation for the reduced nausea the PK data in may provide the information. In terms of AUC the formulation of Example 1 and 3 possess an AUC for both venlafaxine and ODV in the same order as the commercial product, yet causes less nausea, indicating that total exposure is not indicative of adverse incidents. In terms of Cmax EXAMPLE 1, EXAMPLE 3 and the commercial product have values of a similar order although the EFFEXOR XR is greater. This would indicate that the reduction in the nausea exhibited by EXAMPLE 1 and 3 is not solely caused by the reduced Cmax.

## Claims

1. An oral pharmaceutical or veterinary dosage form for extended release of a biologically active drug which is soluble in gastrointestinal media, the dosage form comprising:
(i) a solid core formed from a melt of a first substance which is substantially insoluble in gastrointestinal media and having dispersed therein the active drug, and
(ii) a coating applied to the core by drying or cooling as appropriate from a solution, dispersion or melt, the coating comprising a second substance which is insoluble in gastrointestinal media and a component which is soluble in gastrointestinal media.

2. A dosage form according to Claim 1 wherein the core comprises from about 25% to about 75% (w/w) of the first substance.

3. A dosage form according to Claim 1 or Claim 2 wherein the core comprises from about 75% to about 25% (w/w) of the active drug.

4. A dosage form according to any one of Claims 1 to 3 wherein the core comprises from 0 to 50% (w/w) of filler or excipient comprising diluent, glidant or lubricant or any combination thereof.

5. A dosage form according to any one of Claims 1 to 4 wherein the first substance in the core is a hydrophobic wax.

6. A dosage form according to any one of Claims 1 to 5 wherein the coating substance is a hydrophobic wax.

7. A dosage form according to Claim 5 or Claim 6 wherein the wax is selected from one or more of stearic acid, hydrogenated vegetable oil, glyceryl monostearate or cetostearyl alcohol.

8. A dosage form according to any one of claims 1 to 7 wherein the soluble component is a polyethylene glycol.

9. A dosage form according to any one of claims 1 to 7 wherein the soluble component in the coating is an active drug.

10. A dosage form according to claim 9 wherein the active drug is the same as the drug in the core.

11. A dosage form according to any one of claims 1 to 10 wherein the active drug is venlafaxine hydrochloride.

12. A dosage form according to any one of Claims 1 to 11 wherein the ratio of second substance to soluble component is from about 12:1 (w/w) to about 1:10 (w/w).

13. A dosage form according to any one of Claims 1 to 11 wherein the ratio of second substance to soluble component is from about 6:1 (w/w) to 4:1 (w/w).

14. A form according to any one of Claims 1 to 11 wherein the ratio of second substance to soluble component is about 5:1 (w/w).

15. A dosage form according to any one of Claims 1 to 14 wherein the core comprises a diluent.

16. A dosage form according to Claim 15 wherein the diluent is microcrystalline cellulose.

17. A pharmaceutical dosage form according to any one of Claims 1 to 16 wherein the core comprises a glidant.

18. A pharmaceutical dosage form according to Claim 17 wherein the glidant is silicon dioxide.

19. A pharmaceutical dosage form according to any one of Claims 1 to 18 wherein the core comprises a lubricant.

20. A pharmaceutical dosage form according to Claim 19 wherein the lubricant is magnesium stearate.

21. A process for the preparation of a pharmaceutical dosage form as claimed in any one of Claims 1 to 17 which comprises:
a) melting a mixture of the first insoluble substance and the active drug and solidifying to provide a core,
b) spray coating the core with a solution of the second substance and the soluble component or hot melt coating a melt of the second substance containing a dispersion of the soluble component; and
c) drying if appropriate.
